# NEW EUROPEAN PATENT SPECIFICATION

(11) **EP 0 194 276 B2**
(45) Date of publication and mention of the opposition decision: **16.01.2002**
(45) Mention of the grant of the patent: 11.08.1993
(21) Application number: 85904274.9
(22) Date of filing: 03.09.1985
(51) Int. Cl.: C12N 15/13, C12N 15/62, A61K 39/395, C12P 21/00, G01N 33/563

(54) **PRODUCTION OF CHIMERIC ANTIBODIES**
HERSTELLUNG VON CHIMÄREN ANTIKÖRPERN
PRODUCTION D'ANTICORPS CHIMERIQUES

(30) Priority: 03.09.1984 GB 8422238
(43) Date of publication of application: 17.09.1986
(73) Proprietor: CELLTECH THERAPEUTICS LIMITED, Slough, Berkshire SL1 3WE (GB)
(72) Inventor: NEUBERGER, Michael, Samuel, Cambridge CB2 1MA (GB); RABBITTS, Terence, Howard, Hildersham Cambridge CB1 6BU (GB)
(74) Representative: Mercer, Christopher Paul
(86) International application number: GB8500392
(87) International publication number: WO8601533

(56) References cited:
- EP-A- 0 068 763
- EP-A- 0 090 898
- EP-A- 0 105 521
- EP-A- 0 120 694
- EP-A- 0 125 023
- EP-A- 0 150 126
- WO-A-83/03971
- WO-A-84/00382
- Chemical Abstracts, volume 95, no.4, 27 July 1981, Columbus, Ohio (US) V. Raso et al "Hybrid antibodies with dual specificity for the delivery of ricin to immunoglobulin-bearing target cells", see page 363, abstract 30711m
- Nature, 314, no.6010, April 1985 London (GB) Shun-Ichi Takeda et al "Construction of chimaeric processes immunoglobulin genes containing mouse variable and human constant region sequences", pages 452-45463, abstract 30711m, & Cancer Res. 1981, 41(6), 2073-8
- Nature, 314, no.6008, 21 March 1985, London (GB) M. Neuberger et al "A hapten-specific chimaeric Ig E antibody with human physiological effector function", pages 268-270t region sequences", pages 452-45463, abstract 30711m, & Cancer Res. 1981, 41(6), 2073-8
- Nature, 312, no.5995, December 1984, London (GB) G. Boulianne et al "Production of functional chimaeric mouse/human antibody", pages 643-646ctor function", pages 268-270t region sequences", pages 452-45463, abstract 30711m, & Cancer Res. 1981, 41(6), 2073-8
- Nature, 312, no.5995, December 1984, London (GB) M. Neuberger et al "Recombinant antibodies possessing novel effector functions", pages 604-608
- Nature, vol. 309, 24 May 1984, pages 364-367e, J. Sharon et al.

## Description

The present invention relates to a process for the production of chimeric antibodies using recombinant DNA techniques.

In the present application, the term 'chimeric antibody' is used to describe a protein comprising at least the antigen binding portion of an immunoglobulin molecule (Ig) attached by peptide linkage to at least part of another non-Ig protein.

Sharon J. et al., Nature, 309, 364-367, 24th May 1984 disclose the preparation of a gene encoding the V region of the mouse heavy chain and the C region of a mouse κ light chain. The gene was introduced into a mouse myeloma cell line which secreted a mouse light chain. The transformed cell produced dimeric molecules. It is indicated that structural studies on these dimeric molecules might provide insights into designing antibody molecules consisting of V_{H} and V_{L} attached to other molecules of interest such as enzymes and toxins.

WO-A-84/00382 (US Dept. of Commerce) discloses a number of antibody conjugates for use in the prevention of graft versus host disease. The conjugates are produced by covalently attaching a protein toxin, ricin, to a monoclonal antibody by the use of the chemical linker m-maleimidobenzoyl-N-hydroxy-succinimide ester.

In recent years, advances in molecular biology based on recombinant DNA techniques have provided processes for the production of a wide range of heterologous polypeptides by transformation of host cells with heterologous DNA sequences which code for the production of the desired products.

EP-A-0 088 994 (Schering Corporation) proposed the construction of recombinant DNA vectors comprising a ds DNA sequence which codes for a variable region of a light or a heavy chain of an Ig specific for a predetermined ligand. The ds DNA sequence is provided with initiation and termination codons at its 5'- and 3'- termini respectively, but lacks any nucleotides coding for amino acids superfluous to the variable region. The ds DNA sequence is used to transform bacterial cells. The application does not contemplate the production of chimeric antibodies.

EP-A-0 102 634 (Takeda Chemical Industries Limited) describes the cloning and expression in bacterial host organisms of genes coding for the whole or a part of human IgE heavy chain polypeptide, but does not contemplate the production of chimeric antibodies.

EP-A 0 120 694 (Celltech Ltd.), which was published after the priority date of the present application, describes processes for producing Ig molecules by recombinant DNA technology. It also discloses a process for producing modified Ig molecules in which the antigen-binding region is derived from a first species, class or subclass and other parts of the Ig molecule is derived from a second, different species, class or subclass of Ig.

EP-A-0 125 023 (Genentech Inc. et al.), which was published after the priority date of the present application, proposes the use of recombinant DNA techniques in bacterial cells to produce Ig's which are analogous to those normally found in vertebrate systems and to take advantage of the gene modification techniques proposed therein to construct modified forms of Ig.

It is believed that the proposals set out in the above Genentech application did not lead to the expression of any significant quantities of Ig polypeptide chains, nor to the production of Ig activity, nor to the secretion and assembly of the chains into the desired chimeric Ig's.

The production of monoclonal antibodies was first disclosed by Kohler and Milstein (Kohler, G. and Milstein, C., Nature, 256, 495-497, 1975). Such monoclonal antibodies have found widespread use not only as diagnostic reagents (see, for example, 'Immunology for the 80s, Eds. Voller, A., Bartlett, A., and Bidwell, D., MTP Press, Lancaster, 1981) but also in therapy (see, for example, Ritz, J. and Schlossman, S.F., Blood, 59, 1-11, 1982).

The recent emergence of techniques allowing the stable introduction of Ig gene DNA into myeloma cells (see, for example, Oi, V.T., Morrison, S.L., Herzenberg, L.A. and Berg, P., PNAS USA, 80, 825-829, 1983; Neuberger, M.S., EMBO J., 2, 1373-1378, 1983; and Ochi, T., Hawley, R.G., Hawley, T., Schulman, M.J., Traunecker, A., Kohler, G. and Hozumi, N., PNAS USA, 80, 6351-6355, 1983), has opened up the possibility of using in vitro mutagenesis and DNA transfection to construct recombinant Igs possessing novel properties.

However, it is known that the function of an Ig molecule is dependent on its three dimensional structure, which in turn is dependent on its primary amino acid sequence. Thus, changing the amino acid sequence of an Ig may adversely affect its activity. Moreover, a change in the DNA sequence coding for the Ig may affect the ability of the cell containing the DNA sequence to express, secrete or assemble the Ig.

It is therefore not at all clear that it will be possible to produce functional altered antibodies by recombinant DNA techniques.

Similar considerations apply to other proteins. It therefore cannot be expected that fusion of a gene coding for at least part of an Ig with a gene coding for at least part of a non-Ig protein will lead to expression of any protein, let alone expression of protein which can be secreted and assembled to give a functional chimeric antibody.

However, the present inventors have now discovered unexpectedly that it is possible to produce by recombinant DNA techniques secreted, assembled chimeric antibodies comprising at least part of an Ig molecule and at least part of a non-Ig protein, in which both parts are capable of functional activity.

This surprising result is achieved by the use of the process of the present invention, which comprises:
a) preparing an expression vector including a suitable promoter operably linked to a DNA sequence comprising a first part which encodes at least the variable region of the heavy or light chain of an Ig molecule and a second part which encodes at least part of a second protein;
b) transforming an immortalised mammalian cell line, which secretes an isolated light or heavy chain respectively complementary to the part of the Ig molecule encoded by the first part of the vector prepared in step (a), with the prepared vector; and
c) culturing said transformed cell line to produce a chimeric antibody.

The present invention also provides an alternative process for producing a chimeric antibody comprising at least part of an Ig molecule and at least part of a non-Ig protein, in which both parts are capable of functional activity, which process comprises:
a) preparing a first expression vector including a suitable promoter operably linked to a DNA sequence comprising a first part which encodes at least the variable region of the heavy or light chain of an Ig molecule and a second part which encodes at least part of a non-Ig protein;
b) preparing a second expression vector including a suitable promoter operably linked to a DNA sequence which encodes at least the variable region of a complementary light or heavy chain respectively of an Ig molecule;
c) transforming an immortalised mammalian cell line with the first and second expression vectors prepared in steps a) and b); and
d) culturing said transformed cell line to produce the chimeric antibody.

The present invention further provides another alternative process for producing a chimeric antibody comprising at least part of an Ig molecule and at least part of a non-Ig protein, in which both parts are capable of functional activity, which process comprises:
a) preparing an expression vector including: a first DNA sequence comprising a first part which encodes at least the variable region of the heavy or light chain of an Ig molecule and a second part which encodes at least part of a non-Ig protein operably linked to a suitable promoter; and a second DNA sequence which encodes at least the variable region of a complementary light or heavy chain respectively of an Ig molecule operably linked to a suitable promoter;
b) transforming an immortalised mammalian cell ine with the expression vector prepared in step a); and
c) culturing said transformed cell line to produce the chimeric antibody.

The immortalised cell line is preferably of lymphoid origin, such as a myeloma, hybridoma, trioma or quadroma cell line. The cell line may also comprise a normal lymphoid cell, such as a B-cell, which has been immortalised by transformation with a virus, such as the Epstein-Barr virus. Most preferably, the immortalized cell line is a myeloma cell line or a derivative thereof.

It is known that some immortalised lymphoid cell lines, such as myeloma cell lines, in their normal state secrete isolated Ig light or heavy chains. Such cell lines can be used in the first process of the invention, provided that the normally secreted chain is complementary to the part of the Ig molecule encoded by the first part of the vector prepared in step a).

However, where the immortalised cell line does not secrete or does not secrete a complementary chain, it will be necessary to use the second or third process of the invention.

The techniques by which such vectors can be produced and used to transform the immortalised cell lines are well known in the art, and do not form any part of the invention. However, they are well illustrated in the following Examples.

In the case where the immortalised cell line secretes a complementary light or heavy chain, the transformed cell line may be produced by transforming a suitable bacterial cell with the vector and then fusing the bacterial cell with the immortalised cell line, e.g. by spheroplast fusion.

The first part of the DNA sequence, which encodes at least the variable region of the heavy or light chain of the Ig molecule, may be joined directly to the non-lg protein part thereof. Alternatively, the variable region part may be joined to the non-Ig protein part by an intervening sequence which encodes a specific cleavage sequence, for instance a Factor Xa cleavage sequence as described in EP-A-0 161 937. Reference may be made to this application for further discussion of the use to specific cleavage sequences.

According to a second aspect of the present invention, there is provided a chimeric antibody comprising at least part of an Ig molecule and at least part of a non-Ig protein, in which both parts are capable of functional activity, the antibody comprising:
a) a first polypeptide chain comprising (i) at least the variable region of the heavy or light chain of an Ig molecule and (ii) at least part of a non-Ig protein, the parts (i) and (ii) being linked together by a specifically clearable linker sequence; and
b) a second polypeptide chain comprising at least the variable region of a complementary light or heavy chain respectively of an Ig molecule,
the first and second chains being associated together so as to form an antigen binding site.

In the first polypeptide chain, the non-Ig protein may be:
i) at least the active portion or all of an enzyme;
ii) a protein having a known binding specificity; or
iii) a protein toxin, such as ricin.

The chimeric antibody in case i) above may be used, in particular, in an enzyme linked immunoassay (ELISA) system, in place of the present use of separate antibodies and enzymes.

The chimeric antibody in case ii) above may also be used in immunoassays. For instance, the protein may have a binding specificity for an easily detectable label, such as a heavy or radioactive metal or a dyed or dyeable molecule.

The chimeric antibody in case iii) above will clearly be of use in therapy, for instance as a targeted cytotoxic agent for cancer therapy. In this respect reference may be made to an article by Thorpe et al. (Thorpe, P.E., Edwards, D.C., Davies, A.J.S. and Ross, W.C.J., in 'Monoclonal Antibodies in Clinical Medicine', 167-201, Eds., McMichael, A.J. and Fabre, J.W., Academic Press, 1982).

The chimeric antibodies of the present invention include a specific cleavage site between the variable region part (i) and the non-Ig protein part (ii). Such chimeric antibodies may be used for purifying the non-Ig protein. For instance, the variable region may be made specific for a hapten which can be immobilised on a chromatography medium. The chimeric antibody can then be immobilised by affinity chromatography and contaminating material can be washed away. The non-Ig protein can then be cleaved from the variable region either before or after the variable region is eluted from the chromatography medium.

The present invention is described in more detail, by way of non-limiting illustration only, with reference to the accompanying drawings; in which:
Figure 1A shows the structure of plasmid pSV-Vµ1;
Figure 1B shows the predicted structure of the IgM molecule produced by the J558L cell line transformed with the plasmid pSV-Vµ1;
Figure 2 shows polyacrylamide gels of the purified IgM molecule produced by the J558L cell line transformed with the plasmid pSV-Vµ1;
Figure 3A shows the structure of plasmid pSV-µ_{NPγ}δ;
Figure 3B shows the predicted structure of the F(ab)'₂ chimeric antibody produced by the J558L cell line transformed with the plasmid pSV-V_{NPγ}δ;
Figure 4 shows polyacrylamide gels of purified products produced by the J558L cell line transformed with various plasmids;
Figure 5 shows polyacrylamide gels of purified products produced by the J558L cell line transformed with various plasmids in the presence or absence of tunicamycin;
Figure 6A shows the structure of plasmid pSV-V_{NPγ}SNase;
Figure 6B shows the predicted structure of the chimeric antibody produced by the J558L cell line transformed with the plasmid pSV-V_{NPγ}SNase;
Figure 7 shows polyacrylamide gels used for monitoring SNase activity;
Figure 8 shows an ELISA-type assay;
Figure 9 shows the structure of plasmid pSV-V_{NPγ}myc;
Figure 10 shows an assay for c-myc antigenic determinant in Fab-myc;
Figure 11A shows the structure of plasmid pSV-V_{NPγ}Klenow;
Figure 11B shows the predicted structure of the Fab-γKlenow chimeric antibody produced by the J558L cell line transformed with plasmid pSV-V_{NPγ}Klenow;
Figure 12 shows polyacrylamide gels of the purified Fab-γKlenow chimeric antibody; and
Figure 13 shows an assay for Klenow fragment activity.

In the drawings which show plasmids, thin horizontal lines depict pSV2 gpt vector, thick lines represent mouse or human Ig gene DNA, exons are represented by boxes, and hatched areas represent non-Ig protein DNA.

The locations of the heavy chain locus transcription enhancer element (E) and the gpt gene are indicated. Restriction endonuclease cleavage sites are abbreviated as follows:
R = Eco RI; Xh = Xho I; S = Sac I; and S1/Xh = a sequence formed by joining a Sal I site to an Xho I site. The sequence is presented around the Xho I site of plasmid pSV-V_{NPγ}SNase, which forms a junction of the γ2b CH2 exon and the SNase gene.

In the diagrammatic representations of the predicted structures of Ig molecules and chimeric antibodies, disulphide linkages between heavy (H) and light (λ) chains are indicated by (-S-). Only one subunit of the decavalent pSV-Vµ1 encoded IgM is illustrated.

### PRELIMINARY EXPERIMENTS

In the following Examples use is made of an established mouse plasmacytoma cell line J558L which secretes λ₁ light chains but does not produce any Ig heavy chain. This plasmacytoma cell line is described by Oi et al. (Oi, V.T., Morrison, S.L., Herzenberg, L.A., and Berg, P., PNAS USA, 80, 825-829,1983).

In the Examples, reduced samples were analyzed on 12% polyacrylamide gels, while unreduced samples were analyzed on 7% polyacrylamide gels.

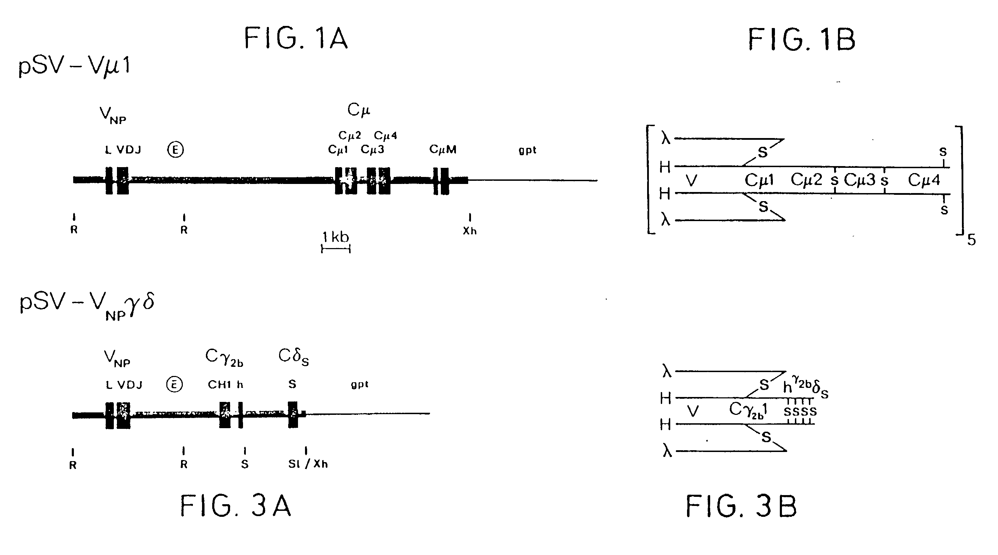

The Examples also use plasmid pSV-Vµ1 described by Neuberger, M.S., EMBO J., 2, 1373-1378, 1983. This plasmid comprises a complete mouse immunoglobulin µ gene cloned into the expression vector pSV2 gpt, and is shown in Figure 1A. The Ig µ polypeptide encoded by this plasmid has a heavy chain variable region, V_{H}, characteristic of λ₁ light chain-bearing mouse antibodies which are specific for the hapten 4-hydroxy-3-nitrophenacetyl (NP): NP binding activity should therefore be formed following association of the pSV-Vµ1 encoded heavy chain with mouse λ₁ light chains.

To confirm this, pSV-Vµ1 DNA was introduced by spheroplast fusion into the J558L cell line and growth in selective medium essentially as described in the Neuberger article referred to above, except that HAT was omitted from the selective medium and mycophenolic acid was used at 5µg/ml, as described in the Oi et al. article referred to above. Cells were cloned by limiting dilution. Antibody samples were purified from supernatants of cloned J558L transfectants grown in Dulbecco's modified Eagle's medium containing 5% foetal calf serum. Supernatants (2 litres) were passed over 2ml columns of 4-hydroxy-5-iodo-3-nitrophenacetyl-amino-caproic acid Sepharose (NIPcap-Sepharose) and antibody eluted from the washed sorbent with 1mM NIPcapOH in phosphate buffered saline.

Biosynthetically labelled antibody was purified on 40 µl NIPcap-Sepharose columns from supernatants of cells incubated for 4h at 37°C in medium containing L-[³⁵S]-methionine. Tunicamycin was, if required, included during the labelling and during a 2h preincubation at 8µg/ml (parallel incubations with an IgE secreting cell line confirmed the efficacy of this treatment).

Stably transfected cells were selected and cloned by limiting dilution. Twenty clones were analyzed by radioimmunoassay and each secreted high levels of an NP-specific IgM antibody. As illustrated in Figure 2, homogeneous anti-NP IgM antibody can be purified from supernatants of pSV-Vµ1 transfected clones with a yield of about 3mg/ml.

In Figure 2 : lane (a) shows purified antibody (30µg) which has been boiled with 2-mercaptoethanol prior to electrophoresis and stained with Coomassie blue; and lane (b) shows markers to enable the molecular weight of the chains to be estimated. The predicted structure of the Ig molecule isolated from pV-Vµ1 transfected clones is shown in Figure 1B.

### F(ab)'₂ - like modified antibody

A derivative of pSV-Vµ1 was constructed in which the Cµ exons were replaced by the CH1 and hinge exons of the mouse γ2b gene. To provide translation termination and polyadenylation sequences, an exon, Cδs, derived from the gene encoding secreted mouse δ chains was placed at the 3' end of the gene. The constructed plasmid pSV-V_{NPγ}δ is shown in Figure 3A.

To construct plasmid pSV-V_{NPγ}δ, the V_{NP} exons from pSV-Vµ1 contained on a common Eco RI fragment are placed in a vector consisting of the Bam HI - Eco RI fragment of pSV2gpt (see Mulligan, R.C. and Berg, P., PNAS USA, 78, 2072-2076, 1981) with an Xho I adapter in the Bam HI site. The plasmid contains an Eco RI - Sac I mouse Cγ2b fragment derived from phage λMYG9 (see Neuberger, M.S. and Calabi, F., Nature, 305, 240-243, 1983). The mouse Cδₛ exon as the PSV-V_{NPγ}δ plasmid is contained in a Bam HI fragment of phage Ch 257 3 (see Cheng, H. - L., Blattner, F. R., Fitzmaurice, L., Mushinski, J.F., and Tucker, P.W., Nature, 296, 410-415, 1982) which was obtained as a Sac I - Sal I fragment after cloning in M13mp 11.

The truncated heavy chain gene of plasmid pSV-V_{NPγ}δ would be expected to direct the synthesis of a F(ab)'₂ -like chimeric antibody, as shown diagrammatically in Figure 3B, consisting of two IgG2b Fab molecules disulphide linked together through the γ2b hinge with a 21 amino acid tail piece at the carboxy terminus encoded by the Cδₛ exon.

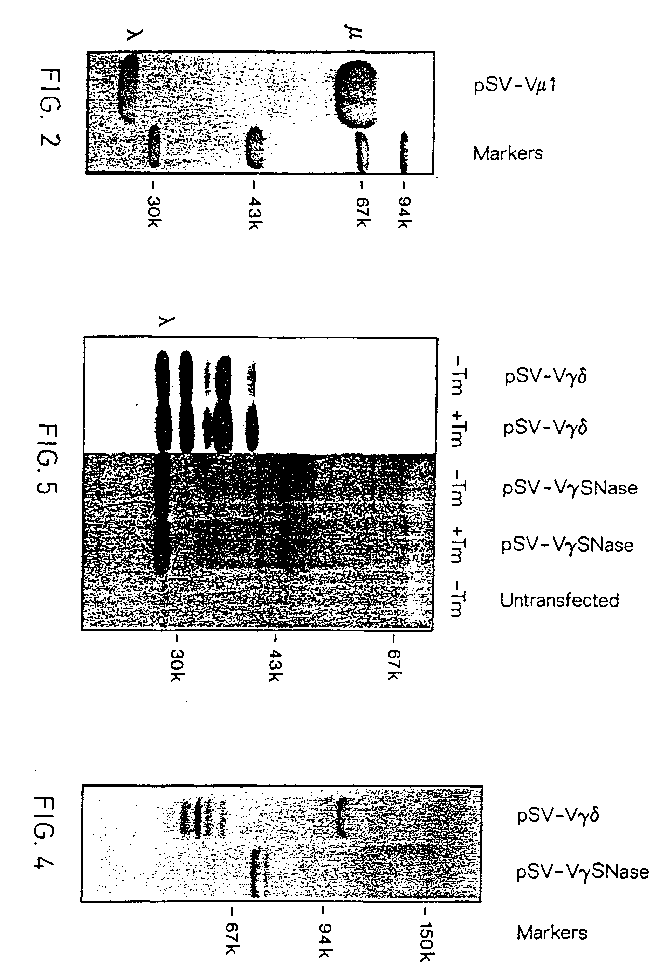

Plasmid pSV-V_{NPγ}δ was transfected into J558L cells and radioimmunoassay revealed that stably transfected cells secreted high levels of λ₁-bearing anti-NP antibody. This NP-specific antibody was purified from culture supernatants of several transfected clones with a yield of 5 to 10mg/l. Polyacrylamide gel electrophoresis of the purified material (Figure 4) shows that the major protein species has an unreduced molecular weight of about 110,000 daltons. After reduction, a band comigrating with λ light chain as well as several higher molecular weight polypeptides are observed. The most abundant of these larger polypeptides has a molecular weight of 31,000 and would constitute the heavy chain of the F(ab)'₂ like antibody. However, there is clear contamination of the pSV-V_{NPγ}δ F(ab)'₂ antibody with other NP-binding material that has an unreduced molecular weight of around 50,000 daltons and is composed of λ₁ light chains and one of several heavy chains in the molecular weight range 36,000 to 40,000. The presence of this minor antibody component does not reflect glycosylation heterogeneity as the electrophoretic mobility of the pSV-V_{NPγ}δ encoded anti-NP antibody is unaffected by inclusion of tunicamycin in the incubation medium during biosynthetic labelling experiments (Figure 5). It is likely that the minor bands differ from the F(ab)'₂ antibody in the carboxy terminal portion of the heavy chain, possibly as a result of alternative processing of pSV-V_{NPγ}δ immunoglobulin gene RNA transcripts. Nevertheless, despite the contaminating bands, it is clear that F(ab)'₂-like anti-NP antibody can be synthesized and secreted in good yield by pSV-V_{NPγ}δ transfected J558L cells.

### Experiments according to the Invention

### Example 1- Fab-nuclease chimeric antibody

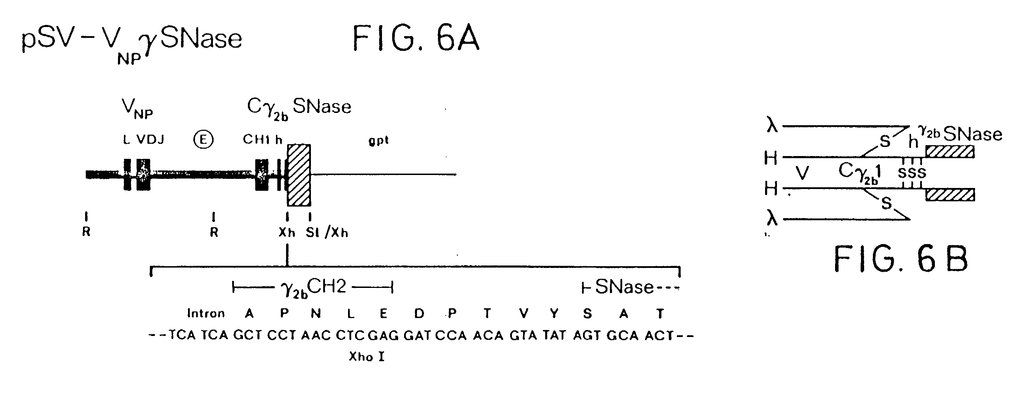

A DNA restriction fragment containing the S. aureus nuclease (SNase) gene was inserted into the Xho I site located in the CH2 exon of the mouse γ2b gene. Plasmid pSV-V_{NPγ}SNase was assembled by inserting the V_{NP} exons contained on the common Eco RI fragment into the vector comprising the Bam HI - Eco RI fragment of pSV2gpt with an Xho I adapter in the Bam HI site as described above. An Eco RI - Xho I mouse Cγ2b fragment derived from phage λMYG9 was also inserted in the vector, so that pSV-V_{NPγ}SNase contains the γ2b CH1, hinge and 5' end of the CH2 exons. The SNase coding region is derived from an M13mp8 clone containing an S. aureus Sau 3A fragment in the Bam HI site. Removal of the SNase gene from M13mp8 as a Bam - Sal I fragment and recloning in M13mp12W (see Karn, J., Mathes, H. W. D., Gait, M. J., and Brenner, S., Gene, 32, 217-224, 1984) allowed its isolation as an Xho I - Sal I fragment for final assembly of the pSV-V_{NPγ}SNase, the structure of which is shown in Figure 6A.

The heavy chain gene of pSV-V_{NPγ}SNase is similar to that of pSV-V_{NPγ}δ except that the Cδₛ exon has been removed and replaced by an exon containing the first four codons of the γ2b CH2 exon fused in phase to the nuclease coding region. SV40-derived sequences of the pSV2gpt-derived vector provide polyadenylation signals.

J558L cells were transfected with pSV-V_{NPγ}SNase and cells surviving in selective medium were cloned by limiting dilution. Radioimmunoassay of supernatants of cloned transfectants revealed that about one third were positive for the production of λ-bearing anti-NP antibody. Positive clones yielded between 1 mg/1 and 10 mg/1 of NP binding antibody, which has the predicted structure shown in Figure 6B.

Analysis of biosynthetically labelled antibody by gel electrophoresis reveals a band comigrating with λ₁ light chain as well as two heavy chain bands of molecular weight 45,000 and 46,000 (Figure 5). The difference between these two heavy chains has not been identified but their mobilities agree well with the predicted mobility of the V_{NPγ}SNase heavy chain. Although the sequence Asn-Asn-Thr is present in SNase, the two V_{NPγ}SNase heavy chain bands are still present in samples purified from supernatants of cells that have been biosynthetically labelled in the presence of tunicamycin (Figure 5). This demonstrates that the difference between the two pSV-V_{NPγ}SNase heavy chains is not due to N-linked glycosylation. The V_{NPγ}SNase antibody appears somewhat more heterogenous on a non-reduced gel, giving bands with the expected mobilities of both the F(ab)'₂-SNase and Fab-SNase (Figure 4). The presence of SNase on the heavy chain carboxy terminus might inhibit disulphide linking of the γ2b hinge regions.

To test for nuclease activity in the V_{NPγ}SNase preparation, samples which had been purified on hapten-Sepharose columns were incubated with single stranded DNA substrate. Digestion of the DNA was monitored following agarose gel electrophoresis as follows. Single stranded M13DNA (2µg) was incubated at 37°C for 30 minutes in 25 mM sodium borate, 250 mM NaCI, 10mM CaCl₂, pH 8.5 (20µl) containing varying amounts of V_{NPγ}SNase chimeric antibody or of purified S. aureus nuclease. The quantities of antibody/enzyme used are given in nanograms. DNA in the samples was then analyzed by ethidium bromide fluorescence after electrophoresis through a 1.2% agarose gel. A Hind III digest of phage λ DNA provides size markers. Ca⁺⁺ dependency of the nuclease activity was confirmed by running incubations in the presence of 40mM MgCl₂, 25mM EGTA.

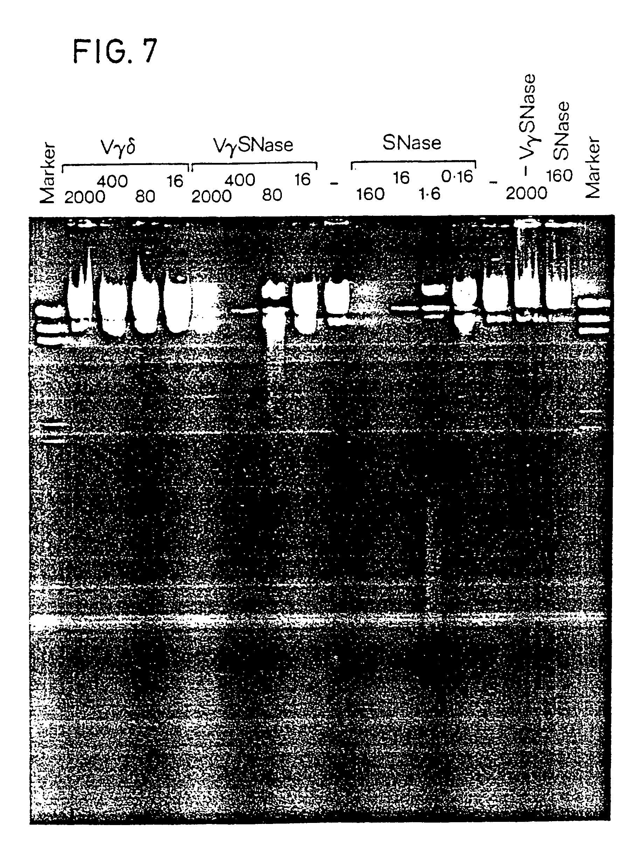

As shown in Figure 7, V_{NPγ}SNase but not V_{NPγ}δ antibodies show nuclease activity and this activity - like that of authentic S. aureus nuclease - is dependent on Ca⁺⁺ but not Mg⁺⁺ ions. As judged on a molar basis, the catalytic activity of the V_{NPγ}SNase sample is about 10% that of authentic S.aureus nuclease.

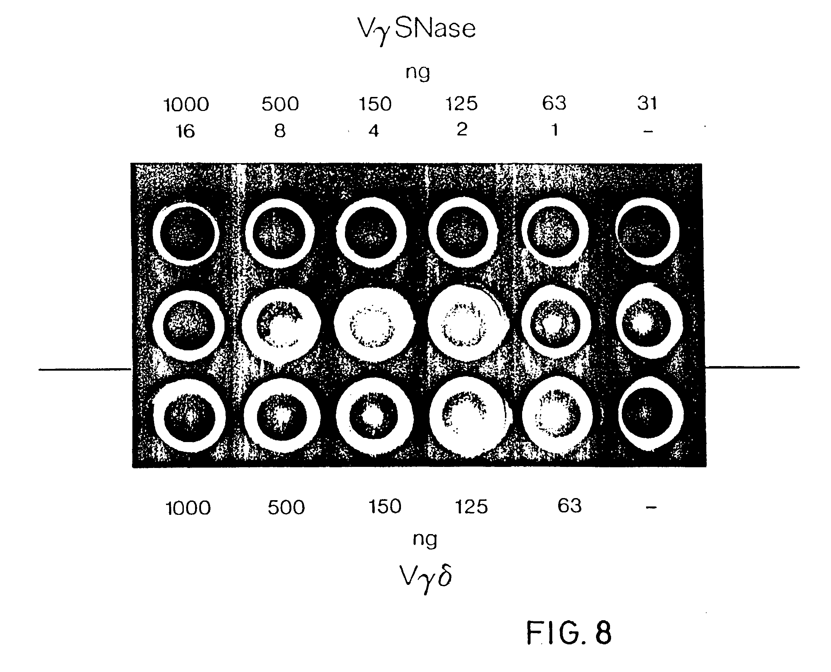

The V_{NPγ}SNase chimeric antibody can be used as a genetically conjugated enzyme linked antibody in ELISA-type assays as shown in Figure 8. Antigen coated plastic plates were incubated with various amounts of V_{NPγ}SNase protein and bound antibody was then detected by virtue of its nuclease activity. This was achieved by addition to the plate of a solution containing DNA and ethidium bromide. Following digestion of the DNA substrate by the immobilized V_{NPγ}SNase antibody, the fluorescence due to the DNA/ethidium bromide complex substantially decreased.

In particular, polyvinyl microtitre plates were coated with (NIP)₂₀ - bovine serum albumin (40µg/ml). After blocking unreacted sites with BSA, dilutions of V_{NPγ}SNase or V_{NPγ}δ chimeric antibodies were incubated in the wells, the amounts of antibody being given in nanograms in Figure 8. After washing off unbound material, a solution (40µl) containing 1µg M13 single stranded DNA and 1µg/ml ethidium bromide in pH 8.5 buffer was added. The plate was photographed after a 1 hour incubation at 37°C.

As shown in Figure 8, quantities in the range of 10 ng of V_{NPγ}SNase antibody are easily detected and no decrease in fluorescence is obtained with the V_{NPγ}δ antibody control. We have found that the assay may be made at least tenfold more sensitive by increasing the incubation time with the DNA substrate.

### Example 2 - Fab-myc chimeric antibody

The carboxyterminal portion of the mouse c-myc gene was fused to the antibody Fab. The product of the c-myc gene is a protein which contains many thiol groups and is normally resident within the cell. There is no reason to believe that the third exon of c-myc on its own will encode a functional protein domain. Thus, if the Fab-myc fusion protein were secreted from the cell it would provide a source of protein for making anti-myc antisera.

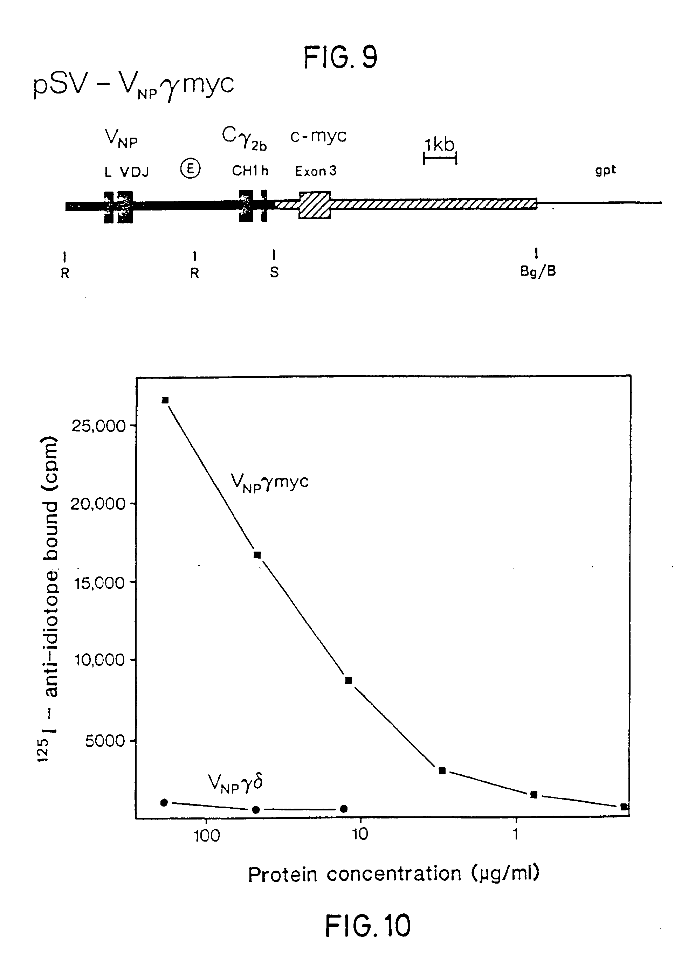

Plasmid pSV-V_{NPγ}myc was assembled essentially as described for the assembly of pSV-V_{NPγ}δ except that the pSV-V_{NPγ}δ Sac I - Xho I fragment containing the Cδₛ exon was replaced by a Sac I - Bgl II fragment containing the 3' exon of mouse c-myc. The restriction site in Figure 9 marked Bg/B is a site formed by joining the Bgl II site at the 3' end of c-myc to the Bam HI site of pSV2gpt. The c- myc fragment comes from phage λMYG2, which contains the translocated c-myc gene of mouse plasmacytoma X63Ag8 (see Neuberger and Calabi, loc. cit).

The plasmid is similar in structure to pSV-V_{NPγ}δ except that the Cδₛ exon is replaced with the 3'-terminal exon of the mouse c-myc gene. This c-myc exon encodes 187 aminoacids (see Bernard, O., Cory, S., Gerondatis, S., Webb, E. and Adams, J. M., EMBO J., 2375-2383, 1983) and should provide the transcription polyadenylation signal.

The plasmid was transfected into J558L and cells from wells positive for production of anti-NP antibody cloned by limiting dilution. Hapten-binding protein was purified from culture supernatants and analyzed for the presence of c-myc antigenic determinants in an indirect radioimmunoassay.

Samples of either the putative Fab-myc or of the anti-NP F(ab)'₂ (to act as control) were incubated in wells of a polyvinyl microtitre plate that had been coated with a monoclonal anti-c-myc antibody; bound anti-NP antibody was then detected using a radioiodinated monoclonal anti-idiotype antibody which recognises the Fab portion of the anti-NP antibodies.

As shown in Figure 10, the Fab-myc clearly binds to the monoclonal anti-c-myc antibody, whereas the anti-NP F(ab)'₂ and other controls do not. The Fab-myc was also recognized by two other monoclonal antibodies that are specific for the carboxyterminal end of c-myc. SDS/polyacrylamide gel electrophoresis of the Fab-myc reveals that it is somewhat heterogenous; a band comigrating with λ₁ light chains and several bands with higher molecular weight in the range 38,000 to 55,000 daltons are observed, without a single dominant heavy chain band being apparent. The expected size of the Fab-myc is 50,000 daltons. It has been observed that, after prolonged storage, precipitates appear in the Fab-myc sample and SDS/polyacrylamide gel electrophoresis reveals more extensive heterogeneity of the heavy chain bands. We therefore believe that the heterogeneity of the Fab-myc protein indicated by the SDS/polyacrylamide gel analysis is most probably due to proteolytic degradation.

### Example 3 - Fab- γ - Klenow Chimeric Antibody.

A plasmid, PSV-V_{NPγ}Klenow, which encodes the heavy chain of a recombinant antibody in which the Klenow fragment of DNA polymerase I is fused to the Fab portion of a mouse IgG2b molecule, was assembled.

The construction of pSV-V_{NPγ}Klenow is analogous to that previously described for pSV-V_{NPγ}SNase. The coding region for the Klenow fragment of DNA polymerase I was obtained as a Bam HI fragment by combining two plasmids, pCJ14 (C.M. Joyce and N.G.D. Grindley, PNAS USA. 80, 1830-1834, 1983) and pCJ89 (C.M. Joyce and N.G.D. Grindley, J. Bacteriol., 158, 636-643, 1984), in which Bam HI linkers have been inserted on either side of the Klenow coding region. This Bam HI fragment was converted into a Sal I fragment by use of linkers and inserted into the unique Xho I site of pSV-V_{NPγ}2b (CH2, CH3).

This antibody/enzyme fusion gene contains a variable region, V_{NP}, such that association of the pSV-V_{NPγ}Klenow heavy chain with the mouse immunoglobulin λ light chain from the J558L cell line will form a binding site for the hapten 4-hydroxy-3-nitrophenacetyl (NP). The V_{NP} gene is linked to exons encoding the CH1, hinge and aminoterminal part of the CH2 domain of a mouse immunoglobulin γ2b heavy chain. The DNA encoding the rest of CH2 and all of CH3 has been replaced by a fragment of the E. coli DNA polymerase I gene that specifies the 5'-3' polymerase and 3'-5' exonuclease activities (the Klenow fragment). This Fab-Klenow fusion gene was cloned into the plasmid vector pSV2gpt which provides a polyadenylation signal for the Fab-Klenow transcription unit and also provides a marker, gpt, that confers resistance to the drug mycophenolic acid and thus allows selection of stably transfected mammalian cells.

Spheroplast fusion was used as a means to introduce pSV-V_{NPγ}Klenow DNA into J558L cells. (The procedure used for protoplast fusion and selection of transfected clones is described in detail by M. S. Neuberger and G. T. Williams in Protein engineering : applications in science, medicine and industry (M. Inouye and R. Sarma, eds.), Academic Press,) Stable transfectants were selected in medium containing mycophenolic acid and the presence of NP-specific antibody in the culture medium of such transfectants was identified by radioimmunoassay. The transfectants were cloned by limiting dilution and one particular clone, JW64/7, that gave a high antibody titre was chosen for further study.

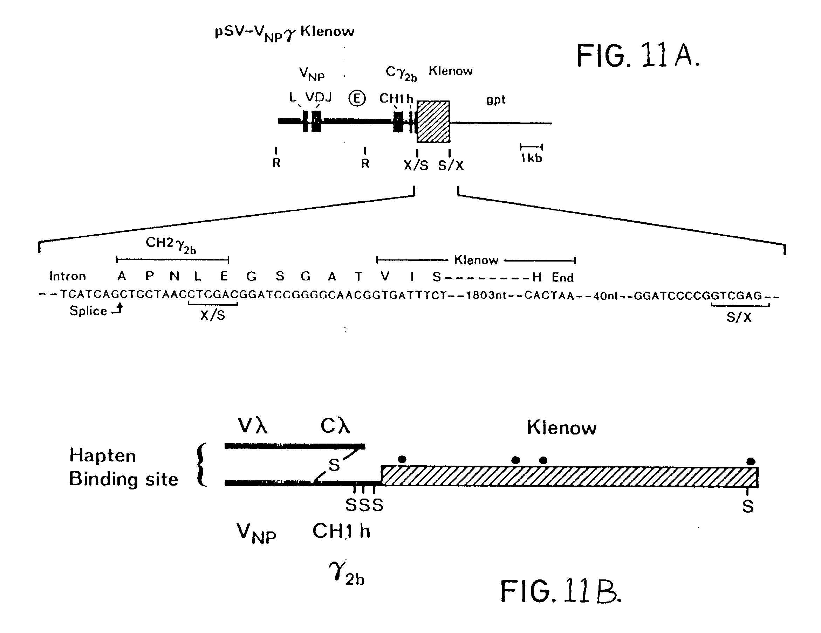

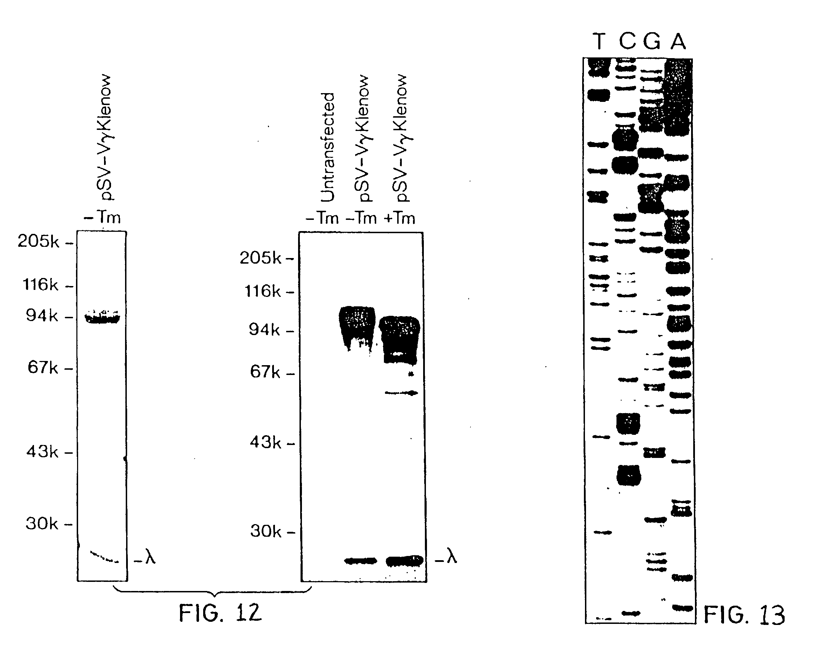

The protein secreted by JW64/7 was examined by SDS/polyacrylamide gel electrophoresis of biosynthetically labelled samples that had been purified on hapten-Sepharose sorbent (Figure 12B). As expected from the predicted structure of the Fab-Klenow protein (Figure 11B), the gel reveals the presence of two polypeptide chains: a band corresponding to λ₁ light chain and a heavy chain of mol. wt. about 96,000 (Figure 12B). Within the Klenow portion of the Fab-Klenow heavy chain, four sequences of the form Asn-X-Thr/Ser are encountered; these might constitute sites for N-linked glycosylation. In order to discover whether the Fab-Klenow heavy chain is in fact glycosylated, biosynthetic labelling experiments were performed in the presence of the glycosylation inhibitor tunicamycin. The results (Figure 12B) reveal that tunicamycin does indeed result in the synthesis of a heavy chain of reduced molecular weight.

Fab-Klenow protein was purified by affinity chromatography on NP-Sepharose from culture supernatant of JW64/7 grown in DMEM/10% foetal calf serum, giving a homogeneous preparation (Figure 12A). The yield varied in the range of 1 to 15 mg of protein per litre of culture supernatant. The 5'-3' polymerase activity of the purified protein was measured using the classical assay (The assay was performed as described by P. Setlow, Methods Enzymol. 29, 3-12, 1974.) in which the enzyme is incubated with "activated" DNA and the four dNTPs, one of which is radiolabelled; the incorporation of radioactivity into acid-precipitable material is followed. In this assay, using activated calf thymus DNA as substrate, the Fab-Klenow gave an activity of about 1.1 x 10³ units/mg as compared to a value of 7 x 10³ units/mg obtained under the same assay conditions using a commercial sample of homogeneous Klenow fragment which had been prepared by proteolytic fragmentation of DNA polymerase I purified from E. coli. A decreased specific activity (expressed as units per mg protein) of the Fab- Klenow compared to the normal enzyme is to be expected in view of their different molecular weights. However, this can only account for part of the difference in activity. Examination of unreduced Fab-Klenow in SDS/polyacrylamide gels (not shown) suggests the presence of divalent F(ab')₂- Klenow as well as monovalent protein. It is possible that the F(ab')2-Klenow might only show half-site reactivity; alternatively, it might be that glycosylation reduces the specific activity of the Fab-Klenow.

A major use of DNA polymerase I Klenow fragment is in the chain termination of DNA sequencing. As shown in Figure 13, Fab-Klenow can indeed be used successfully for this purpose. Figure 13 shows the use of Fab-Klenow in chain termination DNA sequencing. DNA from an M13 clone was sequenced using Fab-Klenow as described by Sanger et al. (F. Sanger, S. Nicklen and A. R. Coulson, PNAS USA, 74, 5463-5476, 1077) using 1 unit per clone of Fab-Klenow.

Thus it has been shown that recombinant antibody technology can usefully be applied to the tagging of specific enzymes such that they are secreted from the cell and can be readily purified to homogeneity in a one step purification.

### CONCLUSION

The above Examples demonstrate that by using the process of the present invention, it is possible to produce secreted, functional chimeric antibodies, which was not previously possible. The process enables the production of a number of chimeric antibodies which have not previously been known.

## Claims

1. A process for the production of a chimeric antibody comprising at least part of an Ig molecule and at least part of a non-Ig protein, in which both parts are capable of functional activity, the process comprising:
a) preparing an expression vector including a suitable promoter operably linked to a DNA sequence comprising a first part which encodes at least the variable region of the heavy or light chain of an Ig molecule and a second part which encodes at least part of a non-Ig protein;
b) transforming an immortalised mammalian cell line, which secretes an isolated Ig light or heavy chain respectively complementary to the part encoded by the first part of the vector prepared in step a), with the prepared vector; and
c) culturing said transformed cell line to produce the chimeric antibody.

2. A process for the production of a chimeric antibody comprising at least part of an Ig molecule and at least part of a non-Ig protein, in which both parts are capable of functional activity, the process comprising:
a) preparing a first expression vector including a suitable promoter operably linked to a DNA sequence comprising a first part which encodes at least the variable region of the heavy or light chain of an Ig molecule and a second part which encodes at least part of a non-Ig protein;
b) preparing a second expression vector including a suitable promoter operably linked to a DNA sequence which encodes at least the variable region of a complementary light or heavy chain respectively of an Ig molecule;
c) transforming an immortalised mammalian cell line with the first and second expression vectors prepared in steps a) and b); and
d) culturing said transformed cell line to produce the chimeric antibody.

3. A process for the production of a chimeric antibody comprising at least part of an Ig molecule and at least part of a non-Ig protein, in which both parts are capable of functional activity, the process comprising:
a) preparing an expression vector including: a first DNA sequence comprising a first part which encodes at least the variable region of the heavy or light chain of an Ig molecule and a second part which encodes at least part of a non-Ig protein operably linked to a suitable promoter; and a second DNA sequence which encodes at least the variable region of a complementary light or heavy chain respectively of an Ig molecule operably linked to a suitable promoter;
b) transforming an immortalised mammalian cell line with the expression prepared in step a); and
c) culturing said transformed cell line to produce the chimeric antibody.

4. The method of any one of claims 1 to 3, wherein the immortalised cell line is of myeloid origin.

5. The method of claim 4, wherein the immortalised cell line is a myeloma cell line or a derivative thereof.

6. The method of claim 1 or any claim dependent thereon wherein transformation is accomplished by transforming a suitable bacterial cell with the vector prepared in step a) and then fusing the bacterial cell with the immortalised cell line.

7. The method of any one of claims 1 to 6, wherein the first part of the DNA sequence, which encodes at least the variable region of the heavy or light chain of the Ig molecule, is directly linked to the second part, which encodes at least part of a non-Ig protein.

8. The method of any one of claims 1 to 6, wherein the first part of the DNA sequence, which encodes at least the variable region of the heavy or light chain of the Ig molecule, is linked to the second part, which encodes at least part of a non-Ig protein, by an intervening sequence which includes a specific cleavage sequence.

9. The method of claim 8, wherein the cleavage sequence is specific for Factor Xa.

10. A chimeric antibody comprising at least part of an Ig molecule and at least part of a non-Ig protein in which both parts are capable of functional activity, the antibody comprising:
a) a first polypeptide chain comprising: (i) at least the variable region of the heavy or light chain of an Ig molecule; and (ii) at least part of a non-Ig protein, the parts (i) and (ii) being linked together by a specifically cleavable linker sequence; and
b) a second polypeptide chain comprising at least the variable region of a complementary light or heavy chain respectively of an Ig molecule,
the first and second chains being associated together so as to form an antigen binding site.

11. The chimeric antibody of claim 10, wherein the non-Ig protein is an enzyme.

12. The chimeric antibody of claim 10, wherein the non-Ig protein is a non-Ig protein of known binding specificity.

13. The chimeric antibody of claim 10, wherein the non-Ig protein is a protein toxin.

14. Use of the chimeric antibody of claim 11 in an ELISA-type assay.

15. Use of the chimeric antibody of claim 12 in an immunoassay.

16. The chimeric antibody of claim 13 for use in therapy.

17. Use of the chimeric antibody of claim 10 in the purification by affinity chromatography of the non-Ig protein part.

## Patentansprüche

1. Ein Verfahren zur Herstellung eines chimärischen Antikörpers, der zumindest einen Teil eines Ig-Moleküls und zumindest einen Teil eines Nicht-Ig-Proteins umfaßt und in dem beide Teile zu funktioneller Aktivität befähigt sind, welches Verfahren folgende Schritte umfaßt:
a) Herstellen eines Expressionsvektors, der einen geeigneten, mit einer DNA-Sequenz operativ verbundenen Promotor enthält, wobei die DNA-Sequenz einen ersten, zumindest die variable Region der schweren oder leichten Kette eines Ig-Moleküls codierenden Teil und einen zweiten, zumindest einen Teil eines Nicht-Ig-Proteins codierenden Teil umfaßt;
b) Transformieren einer immortalisierten Säugetierzellinie mit dem hergestellten Vektor, wobei die Säugetierzellinie eine isolierte Ig-leichte bzw. Ig-schwere Kette ausscheidet, die zu dem Teil, der von dem ersten Teil des in Stufe (a) hergestellten Vektors codiert wird, komplementär ist; und
c) Züchtung dieser transformierten Zellinie zur Herstellung des chimärischen Antikörpers.

2. Ein Verfahren zur Herstellung eines chimärischen Antikörpers, der zumindest einen Teil eines Ig-Moleküls und zumindest einen Teil eines Nicht-Ig-Proteins enthält und in dem beide Teile zu funktioneller Aktivität befähigt sind, welches Verfahren folgende Schritte umfaßt:
a) Herstellen eines ersten Expressionsvektors, der einen geeigneten, mit einer DNA-Sequenz operativ verbundenen Promotor enthält, wobei die DNA-Sequenz einen ersten, zumindest die variable Region der schweren oder leichten Kette eines Ig-Moleküls codierenden Teil und einen zweiten, zumindest einen Teil eines Nicht-Ig-Proteins codierenden Teil enthält;
b) Herstellen eines zweiten Expressionsvektors, der einen geeigneten, mit einer DNA-Sequenz operativ verbundenen Promotor enthält, wobei die DNA-Sequenz zumindest die variable Region einer komplementären leichten bzw. schweren Kette eines Ig-Moleküls codiert;
c) Transformieren einer immortalisierten Säugetierzellinie mit den ersten und zweiten, in den Stufen a) und b) hergestellten Vektoren; und
d) Züchtung dieser transformierten Zellinie zur Herstellung des chimärischen Antikörpers.

3. Ein Verfahren zur Herstellung eines chimärischen Antikörpers, der zumindest einen Teil eines Ig-Moleküls und zumindest einen Teil eines Nicht-Ig-Proteins umfaßt und in dem beide Teile zu funktioneller Aktivität befähigt sind, welches Verfahren folgende Schritte umfaßt:
a) Herstellen eines Expressionsvektors, der enthält: eine erste DNA-Sequenz, die einen ersten, zumindest die variable Region der schweren oder leichten Kette eines Ig-Moleküls codierenden Teil und einen zweiten, zumindest einen Teil eines Nicht-Ig-Proteins codierenden Teil in operativer Verbindung mit einem geeigneten Promotor; und eine zweite DNA-Sequenz, die zumindest die variable Region einer komplementären leichten bzw. schweren Kette eines Ig-Moleküls codiert, in operativer Verbindung mit einem geeigneten Promotor umfaßt;
b) Transformieren einer immortalisierten Säugetierzellinie mit dem in Stufe a) hergestellten Vektor; und
c) Züchtung dieser transformierten Zellinie zur Herstellung des chimärischen Antikörpers.

4. Das Verfahren nach einem der Ansprüche 1 bis 3, bei welchem die immortalisierte Zellinie myeloiden Ursprungs ist.

5. Das Verfahren nach Anspruch 4, bei welchem die immortalisierte Zellinie eine Myelom-Zellinie oder ein Derivat hievon ist.

6. Das Verfahren nach Anspruch 1 oder nach einem beliebigen hievon abhängigen Anspruch, bei welchem die Transformation durch Transformieren einer geeigneten Bakterien-Zellinie mit dem in Stufe a) hergestellten Vektor und anschließende Fusion der Bakterienzelle mit der immortalisierten Zellinie erreicht wird.

7. Das Verfahren nach einem der Ansprüche 1 bis 6, bei welchem der erste Teil der DNA-Sequenz, der zumindest die variable Region der schweren oder leichten Kette des Ig-Moleküls codiert, direkt an den zweiten, zumindest einen Teil eines Nicht-Ig-Proteins codierenden Teil gebunden ist.

8. Das Verfahren nach einem der Ansprüche 1 bis 6, bei welchem
der erste Teil der DNA-Sequenz, der zumindest die variable Region der schweren oder leichten Kette des Ig-Moleküls codiert, an den zweiten, zumindest einen Teil eines Nicht-Ig-Proteins codierenden Teil über eine dazwischenliegende Sequenz, die eine spezifische Spaltungsstelle enthält, gebunden ist.

9. Das Verfahren nach Anspruch 8, bei welchem die Spaltungssequenz spezifisch für Faktor Xa ist.

10. Ein chimärischer Antikörper, der zumindest einen Teil eines Ig-Moleküls und zumindest einen Teil eines Nicht-Ig-Proteins umfaßt, bei welchem beide Teile zu funktioneller Aktivität befähigt sind, wobei der Antikörper umfaßt:
a) eine erste Polypeptidkette, welche umfaßt: (i) zumindest die variable Region der schweren oder leichten Kette eines Ig-Moleküls; und (ii) zumindest einen Teil eines Nicht-Ig-Proteins, wobei die Teile (i) und (ii) über eine spezifisch spaltbare Linker-Sequenz miteinander verbunden sind; und
b) eine zweite Polypeptidkette, die zumindest die variable Region einer komplementären leichten bzw. schweren Kette eines Ig-Moleküls umfaßt,
wobei die ersten und zweiten Ketten miteinander assoziiert sind, um eine Antigenbindungsstelle zu bilden.

11. Der chimärische Antikörper nach Anspruch 10, in welchem das Nicht-Ig-Protein ein Enzym ist.

12. Der chimärische Antikörper nach Anspruch 10, in welchem das Nicht-Ig-Protein ein Nicht-Ig-Protein mit bekannter Bindungsspezifität ist.

13. Der chimärische Antikörper nach Anspruch 10, in welchem das Nicht-Ig-Protein ein Protein-Toxin ist.

14. Verwendung des chimärischen Antikörpern nach Anspruch 11 in einem Assay vom ELISA-Typ.

15. Verwendung des chimärischen Antikörpers nach Anspruch 12 in einem Immunoassay.

16. Der chimärische Antikörper nach Anspruch 13 zur Verwendung in der Therapie.

17. Verwendung des chimärischen Antikörpers nach Anspruch 10 bei der Reinigung des Nicht-Ig-Protein-Teils durch Affinitätschromatographie.

## Revendications

1. Un procédé pour la production d'un anticorps chimère ou chimérique comprenant au moins une partie d'une molécule d'Ig (immunoglobuline) et au moins une partie d'une protéine de non-Ig, dans lequel les deux parties sont capables d'une activité fonctionnelle, ce procédé comprenant:
a) la préparation d'un vecteur d'expression comprenant un promoteur convenable relié d'une manière opérationnelle à une séquence d'ADN comprenant une première partie qui code pour au moins la région variable de la chaîne, lourde ou légère, d'une molécule d'Ig et une seconde partie qui code pour au moins une partie d'une protéine de non-Ig;
b) la transformation d'une lignée cellulaire immortalisée de mammifère, qui secrète une chaîne, légère ou lourde, respectivement, d'Ig isolée, complémentaire de la partie codée par la première partie du vecteur préparé dans la phase a), avec le vecteur préparé; et
c) la culture de ladite lignée cellulaire transformée pour produire l'anticorps chimérique.

2. Un procédé pour la production d'un anticorps chimère ou chimérique comprenant au moins une partie d'une molécule d'Ig (immunoglobuline) et au moins une partie d'une protéine de non-Ig, dans lequel les deux parties sont capables d'une activité fonctionnelle, ce procédé comprenant;
a) la préparation d'un premier vecteur d'expression comprenant un promoteur convenable relié d'une manière opératoire à une première séquence d'ADN comprenant une première partie qui code pour au moins la région variable de la chaîne, lourde ou légère, d'une molécule d'Ig et une seconde partie qui code pour au moins une partie d'une protéine de non-Ig;
b) la préparation d'un second vecteur d'expression comprenant un promoteur convenable relié d'une manière opérationnelle à une séquence d'ADN qui code pour au moins la région variable d'une chaîne complémentaire, légère ou lourde, respectivement, d'une molécule d'Ig;
c) la transformation d'une lignée cellulaire immortalisée de mammifère avec les premier et second vecteurs d'expression préparés dans les phases a) et b); et
d) la culture de ladite lignée cellulaire transformée pour produire l'anticorps chimérique.

3. Un procédé pour la production d'un anticorps chimère ou chimérique comprenant au moins une partie d'une molécule de Ig (immunoglobuline) et au moins une partie d'une protéine de non-Ig, dans lequel les deux parties sont capables d'une activité fonctionnelle, ce procédé comprenant:
a) la préparation d'un vecteur d'expression qui comporte : une première séquence d'ADN comprenant une première partie qui code pour au moins la région variable de la chaîne, lourde ou légère, d'une molécule d'lg et une seconde partie qui code pour au moins une partie d'une protéine de non-Ig connectée d'une manière opérationnelle à un promoteur convenable; et une seconde séquence d'ADN qui code pour au moins la région variable d'une chaîne complémentaire, légère ou lourde, respectivement, d'une molécule d'lg reliée d'une manière opérationnelle à un promoteur convenable;
b) la transformation d'une lignée cellulaire immortalisée de mammifère avec le vecteur d'expression préparé dans la phase a); et
c) la culture de ladite lignée cellulaire transformée pour produire l'anticorps chimérique.

4. La méthode de l'une quelconque des revendications 1 à 3, dans laquelle la lignée cellulaire immortalisée est d'origine myéloïde.

5. La méthode de la revendication 4, dans laquelle la lignée cellulaire immortalisée est une lignée cellulaire de myélome ou est dérivée de celle-ci.

6. La méthode de la revendication 1 ou toute revendication dépendant de celle-ci, dans laquelle la transformation est réalisée par transformation d'une cellule bactérienne convenable au moyen du vecteur préparé dans la phase a) et ensuite par fusion de la cellule bactérienne avec la lignée cellulaire immortalisée.

7. La méthode de l'une quelconque des revendications 1 à 6, dans laquelle la première partie de la séquence d'ADN, qui code pour au moins la région variable de la chaîne, lourde ou légère, de la molécule d'lg, est reliée directement à la seconde partie, qui code pour au moins une partie d'une protéine de non-Ig.

8. La méthode de l'une quelconque des revendications 1 à 6, dans laquelle la première partie de la séquence d'ADN, qui code pour, au moins la région variable de la chaîne, lourde ou légère, de la molécule d'Ig, est reliée à la seconde partie, qui code pour au moins une partie d'une protéine de non-Ig, par une séquence intervenante qui comprend une séquence de coupure spécifique.

9. La méthode de la revendication 8, dans laquelle lu séquence de coupure est spécifique pour le Facteur Xa.

10. Un anticorps chimérique comprenant au moins une partie d'une molécule d'Ig et au moins une partie d'une protéine de non-Ig dans laquelle les deux parties sont capables d'activité fonctionnelle, cet anticorps comprenant:
a) une première chaîne polypeptidique comprenant: (i) au moins la région variable de la chaîne, lourde ou légère, d'une molécule d'Ig; et (ii) au moins une partie d'une protéine de non-Ig, les portions (i) et (ii) étant reliées l'une à l'autre par une séquence de liaison spécifiquement coupable ; et
b) une seconde chaîne polypeptidique comprenant au moins la région variable d'une chaîne complémentaire, légère ou lourde, respectivement, d'une molécule d'lg,
les première et seconde chaînes étant associées ensemble pour former un site de liaison d'antigène.

11. L'anticorps chimérique de la revendication 10, dans lequel la protéine de non-Ig est un enzyme.

12. L'anticorps chimérique de la revendication 10, dans laquelle la protéine de non-Ig est une protéine de non-Ig de spécificité de liaison connue.

13. L'anticorps chimérique de la revendication 10, dans lequel la protéine de non-Ig est une toxine protéinique.

14. Utilisation de l'anticorps chimérique de la revendication 11 dans un test de type ELISA.

15. Utilisation de l'anticorps chimérique de la revendication 12 dans un test d'immunité.

16. L'anticorps chimérique de la revendication 13 pour utilisation en thérapie.

17. Utilisation de l'anticorps chimérique de la revendication 10, dans la purification, par chromatographie par affinité, de la partie de protéine de non-Ig.
